# EUROPÄISCHE PATENTSCHRIFT

(11) **EP 2 019 816 B1**
(45) Veröffentlichungstag und Bekanntmachung des Hinweises auf die Patenterteilung: **09.01.2019**
(21) Anmeldenummer: 07729007.0
(22) Anmeldetag: 11.05.2007
(51) Int. Cl.: C07C 45/27, C07C 45/38, B01J 12/00

(54) **TEMPERIERUNG BEI DER DURCHFÜHRUNG VON OXIDATIONSREAKTIONEN VON KOHLENWASSERSTOFFEN**
TEMPERATURE ADJUSTMENT IN OXIDATION REACTIONS OF HYDROCARBONS
TEMPÉRATION LORS DE LA RÉALISATION DE RÉACTIONS D'OXYDATION D'HYDROCARBURES

(30) Priorität: 17.05.2006 EP 06114093
(43) Veröffentlichungstag der Anmeldung: 04.02.2009
(73) Patentinhaber: BASF SE, 67056 Ludwigshafen am Rhein (DE)
(72) Erfinder: VON WATZDORF, Jobst Rüdiger, 68163 Mannheim (DE); KOPPENHÖFER, Gerhard, 67354 Römerberg (DE); KANTHER, Wolfgang, 67125 Dannstadt-Schauernheim (DE); KRUG, Thomas, 67550 Worms (DE); JOHANN, Thorsten, 67063 Ludwigshafen (DE)
(86) Internationale Anmeldenummer: PCT/EP2007/054555
(87) Internationale Veröffentlichungsnummer: WO 2007/131949

(56) Entgegenhaltungen:
- DE-A1- 1 923 048
- DE-A1- 2 737 894
- US-A- 5 840 932

## Beschreibung

Die vorliegende Erfindung bezieht sich auf ein Verfahren zur Durchführung von katalysierten Oxidationsreaktionen von Kohlenwasserstoffen, insbesondere auf die Herstellung von vicinalen Dioxoverbindungen aus den entsprechenden Diolen und auf einen Reaktor, umfassend eine Vorrichtung zur Temperatureinstellung im Ausgangsbereich des Reaktors.

Für Verfahren, die eine katalysierte Oxidationsreaktion in der Gasphase umfassen, beispielsweise die Herstellung von Glyoxal, ist aus EP 1 169 119 der Einsatz von Rohrbündelreaktoren bekannt. Hierbei wird das Gasgemisch in die Reaktionsrohre eingeleitet, in welchen ein Festbett eines katalytisch aktiven Multimetalloxids angeordnet ist. Durch den zwischen dem obersten und dem untersten Rohrboden befindlichen, die Reaktionsrohre umgebenden Raum, wird ein Wärmeaustauschmittelkreislauf geleitet, um Reaktionswärme zu- bzw. abzuführen.

Als Wärmeaustauschmittel sind Temperiermedien bekannt, die im Bereich der herrschenden Reaktionstemperaturen flüssig sind, insbesondere werden Schmelzen von Salzen verwendet.

Aus DE 1 923 048 ist die Herstellung von Glyoxal durch Oxidation der Hydroxyverbindung Ethylenglykol an einem Katalysator bekannt. Als geeignete Oxidationskatalysatoren werden solche genannt, die durch Oxidation einer Kupfer-Zinn-Legierung, einer Kupfer-Zinn-Phosphor-Legierung oder einer Kupfer-Phosphor-Legierung erhalten werden. Die Oxidation findet in der Gasphase statt, wobei eine Hydroxyverbindung, beispielsweise in einem Verdampfer, in die Gasphase überführtes Ethylenglykol mit einem sauerstoffhaltigen Gas, beispielsweise Luft, in Gegenwart des Oxidationskatalysators bei erhöhter Temperatur reagiert. Dem Gasgemisch kann ein Verdünnungsgas, zum Beispiel Stickstoff, Kohlendioxid, Wasserdampf oder ein anderes Gas, welches gegenüber den Reaktionsteilnehmern und den Produkten unter den Reaktionsbedingungen inert ist, zugemischt werden. Bei der Herstellung von Glyoxal aus Ethylenglykol herrschen typischerweise Reaktionstemperaturen von 300 bis 450°C.

Als nachteilig bei dem beschriebenen Verfahren hat sich erwiesen, dass die erreichten Ausbeuten an Glyoxal mit 65 bis 70 %, bezogen auf die eingesetzte Menge an Ethylenglykol, vergleichsweise gering ist.

Hierfür können Nebenreaktionen, insbesondere in den nachgeschalteten Rohrleitungen und Apparaten, welche sowohl homogene Gasphasenreaktionen als auch heterogen katalysierte Wandreaktionen sein können, die zu einem Ausbeuteverlust an Glyoxal führen, verantwortlich sein. Diese unselektiven Oxidationsreaktionen finden verstärkt bei den hohen Temperaturen, wie sie im Austrittsbereich des Glyoxal enthaltenden Produktgasstroms an den Reaktionsrohren herrschen, statt.

Aus US 5,840,932 ist bei einem Verfahren zur Herstellung von Ethylenoxid durch katalytische Oxidation in einem Rohrbündelreaktor bekannt, in einen Bereich unterhalb der Rohre einen gekühlten, aus dem Produktstrom aufbereiteten Teilstrom einzuleiten und diesen mit dem austretenden Produktstrom zu vermischen, mit der Zielsetzung, unmittelbar im Austrittsbereich den Produktstrom zu kühlen. Die Einleitung erfolgt über ein nicht näher spezifiziertes Düsensystem.

Aus DE 27 37 894 ist bekannt, zur intensiven Kühlung eines Produktgasstromes bei der Herstellung von Maleinsäureanhydrid in einem Reaktionsgefäß ein Wärmeaustausch mit einer in Kühlschlangen strömenden Kühlflüssigkeit oder eine Temperaturreduzierung durch Mischen mit einem Kühlgas, welches auch ein Teil des Reaktorrückführgases, ein Teil eines sauerstoffhaltigen Gases oder ein Teil des Produktstromes sein kann, herbeizuführen. Die Einleitung des Kühlgases kann über ein Gasverteiler-System in Form einer Sprinklereinrichtung erfolgen, welche im unteren Bereich des Reaktionsgefäßes, d.h. in der Reaktionsgefäßhaube, angeordnet ist. Hierbei wirkt sich nachteilig der hohe Platzbedarf eines derartigen Gasverteiler-Systems in der Reaktionsgefäßhaube aus, wodurch die Kosten des Reaktionsgefäßes ansteigen können. Als problematisch erweisen sich aber auch die von kaltem Gas durchströmten Einrichtungen, an denen es zu Kondensations- und/oder Desublimationsvorgängen kommen kann. Die damit einhergehenden Beläge auf den Einrichtungen können sich von diesen ablösen und werden in nachfolgende Anlagenteile mitgerissen.

Am Beispiel der Herstellung von Glyoxal zeigt sich trotz eines hohen bis nahezu vollständigen Umsatzes des Eduktes Monoethylenglykol, dass die Ausbeuten an Glyoxal bezogen auf das eingesetzte Monoethylenglykol gering sind. Insbesondere leiten die hohen Temperaturen, die in dem Produktgasstrom herrschen, in den auf den Rohrbündelreaktor folgenden Leitungen und Apparaten eine homogene und/oder heterogene Reaktion des Glyoxals ein, welche zu deutlichen Ausbeuteverlusten führen kann.

Aufgabe der vorliegenden Erfindung ist es daher, ein Verfahrenskonzept und eine entsprechende Vorrichtung bereitzustellen, wodurch bei der Durchführung von katalysierten Oxidationsreaktionen von Kohlenwasserstoffen, insbesondere bei der Herstellung von vicinalen Dioxoverbindungen aus entsprechenden Diolen, beispielsweise die Herstellung von Glyoxal aus dem Edukt Monoethylenglykol, verbesserte Ausbeuten erzielt werden.

Die Lösung der Aufgabe geht aus von einem Verfahren zur Durchführung von katalysierten Oxidationsreaktionen von Kohlenwasserstoffen an einem in einem Reaktor angeordneten Katalysator, wie der Herstellung von vicinalen Dioxoverbindungen aus entsprechenden vicinalen Diolen durch katalysierte Oxydehydrierungsreaktion der Diole mit Luftsauerstoff an einem in den Reaktionsrohren eines Rohrbündelreaktors angeordneten Katalysatorfestbett. Es ist gekennzeichnet dadurch, dass der Produktgasstrom der Oxidationsreaktion unmittelbar nach Austritt aus der Reaktionszone des Reaktors durch Einspeisen eines temperierten Gasstroms gekühlt wird.

Eine Temperierung eines Produktgasstroms unmittelbar nach Austritt aus der Reaktionszone ist überall dort anwendbar, wo ein unter den herrschenden Bedingungen thermisch instabiles Produkt vorhanden ist, welches durch Abkühlung mit einem temperierten Gasstrom stabilisiert werden kann. Die Reaktionszone umfasst einen Bereich eines Reaktors, in dem ein Katalysator beispielsweise als Wirbelbett oder als Festbett in Reaktionsrohren eines Rohrbündelreaktors angeordnet ist.

Das erfindungsgemäße Verfahren wird im Hinblick auf die Herstellung von vicinalen Dioxoverbindungen in einem Rohrbündelreaktor dargestellt.

Das Verfahren zur Herstellung von vicinalen Dioxoverbindungen umfasst das Bereitstellen eines Eduktgemisches, welches insbesondere die entsprechenden vicinalen Diolen umfasst. Im Falle der Herstellung von Glyoxal durch eine katalysierte Oxydehydrierung von Monoethylenglykol wird dieses in einem geeigneten Verdampfer in die Gasphase überführt. Das gasförmige Monoethylenglykol wird einem Kreisgas zugemischt, welches inert gegenüber den Reaktionsteilnehmern bei den herrschenden Reaktionsbedingungen und gegenüber den Produkten ist. Das Kreisgas kann im Wesentlichen Stickstoff und Anteile von Sauerstoff, Kohlendioxid, Kohlenmonoxid und Wasser enthalten. Beispielsweise weist das Kreisgases 0 bis 5 Vol.-% O₂, 0 bis 10 Vol.-% CO₂, 0 bis 5 Vol.-% CO, 0 bis 15 Vol.-% H₂O und eine dem Rest entsprechende Menge Stickstoff auf. Aber auch weitere Bestandteile des Kreisgases sind möglich. Der für die Oxydehydrierung der vicinalen Diolen benötigte Sauerstoff kann durch Zumischung von Frischluft zu dem mit vicinalen Diolen gesättigtem Kreisgas bereitgestellt werden.

Die molaren Verhältnisse an Sauerstoff zu Ethylenglykol vor Eintritt in den Rohrbündelreaktor betragen typischerweise < 1,5 mol Sauerstoff pro mol Ethylenglykol.

Der derart hergestellte Eduktgasstrom wird in einen Rohrbündelreaktor eingeleitet und gelangt in eine Vielzahl von Katalysatorfestbett enthaltenden Reaktionsrohre. Vorzugsweise handelt es sich um einen mit Phosphor dotierten Kupferkatalysator, aber auch der Einsatz von Silberkatalysatoren, die mit Gold, Platin, Rhodium oder Palladium dotiert sind, Silberkatalysatoren mit Kupfer oder Katalysatoren auf Molybdän-Oxid-Basis ist möglich.

Die bei der katalytischen Oxydehydrierung entstehende Reaktionswärme wird durch ein die Reaktionsrohre umgebendes Wärmeaustauschmittel, vorzugsweise in Form eines Salzkreislaufes und einem System aus Dampferzeuger und Dampfüberhitzer, abgeführt. Somit kann die abgeführte Reaktionswärme, beispielsweise zur Erzeugung von Prozessdampf genutzt werden. Üblicherweise ist der verwendete Rohrbündelreaktor einzonig ausgeführt, aber auch eine mehrzonige Ausführung, mit mehreren hintereinander angeordneten Reaktionszonen, in denen jeweils unterschiedliche Temperaturen durch einen separaten Wärmeaustauschkreislauf eingestellt werden können, ist möglich.

Der verwendete Rohrbündelreaktor umfasst weiterhin einen zylindrischen Bereich, welcher an beiden Enden üblicher Weise durch Hauben abgeschlossen ist. In dem zylindrischen Bereich sind eine Vielzahl von Reaktionsrohren zwischen einem obersten und einem untersten Rohrboden angeordnet. Typische Durchmesser des zylindrischen Bereichs liegen bei 2,5 bis 5 m. Rohrbündelreaktoren mit einem derartigen Durchmesser weisen im Allgemeinen 1.000 bis 15.000 Reaktionsrohre, bevorzugt 2.000 bis 10.000 Reaktionsrohre auf. Üblicherweise liegt der Innendurchmesser der Reaktionsrohre bei 20 bis 70 mm, bevorzugt bei 40 bis 60 mm. Die typische Länge der Reaktionsrohre und damit die Länge des zylindrischen Bereichs des Reaktors liegen im Bereich von 1,5 bis 5 m, bevorzugt 2 bis 3,5 m.

Bei der Herstellung von Glyoxal hat sich als vorteilhaft für die angestrebte möglichst vollständige Umsetzung des eingesetzten Monoethylenglykols am Katalysator eine Eintrittstemperatur am Salzkreislauf von 360 bis 390 °C erwiesen. Die Temperatur des die Reaktionsrohre verlassenden Produktgasstroms liegt typischerweise bei 350 bis 370 °C. Anschließend kann der den Rohrbündelreaktor verlassende Produktgasstrom in einem Kreisgasrekuperator abgekühlt werden, und in einem folgenden Quenchschritt können die kondensierbaren Komponenten, welche im Wesentlichen Glyoxal, sowie Nebenprodukte wie Formaldehyd, Glykolaldehyd, Ameisensäure und weitere umfassen, auskondensiert werden. Es folgen weitere Aufarbeitungsschritte, die letztlich zu einem gereinigten Glyoxal führen.

Das erfindungsgemäße Verfahren sieht eine Temperaturreduzierung des Produktgasstroms unmittelbar im Austrittsbereich aus der Reaktionszone vor. Bei einem Rohrbündelreaktor ist dies der Bereich der Reaktorhaube unterhalb der Austrittsöffnung der Reaktionsrohre, so dass die unerwünschten Nebenreaktionen des Produktes weitestgehend unterdrückt werden können, welche für einen Ausbeuteverlust mitverantwortlich sind.

Hierfür ist erfindungsgemäß eine Einspeiseeinrichtung im Ausgangsbereich des Reaktors vorgesehen, im Allgemeinen in der unteren Haube, unmittelbar unterhalb der Austrittsöffnungen der Reaktionsrohre im Bereich zwischen dem untersten Rohrboden und dem Auslass des Rohrbündelreaktors. Die erfindungsgemäße Einspeiseeinrichtung ermöglicht ein Einspeisen eines temperierten Gasstroms, welcher durch intensive Mischung mit dem Produktgasstrom die herrschende Temperatur reduziert.

Als eingespeister temperierter Gasstrom kann ein kaltes, inertes Gas, bevorzugt ein Teilstrom des gekühlten Kreisgases verwendet werden. Der Gasstrom wird vorzugsweise in einem Volumenverhältnis von 1:20 bis 8:10 bezogen auf den Produktgasstrom, insbesondere im Verhältnis 1:10 bis 1:5 eingespeist.

Um eine ausreichende Kühlung des Produktgasstromes zu erreichen, wodurch in geeigneter Weise die unerwünschten Nebenreaktionen unterdrückt werden, liegt die Temperatur des eingespeisten temperierten Gasstromes im Allgemeinen bei 30 bis 300°C, bevorzugt bei 80 bis 200°C und besonders bevorzugt bei 80 bis 120°C. Bei der Abkühlung des Produktgasstromes ist auch der Wärmeverbund zur Kreisgasvorwärmung zu berücksichtigen, so dass es in Abhängigkeit vom eingesetzten Verfahren eine untere Temperaturgrenze gibt. Am Beispiel der Herstellung von Glyoxal hat sich eine Abkühlung von 30 bis 60 °C als geeignet erwiesen, wobei die untere Temperaturgrenze bei in etwa 300 °C liegt.

In dem erfindungsgemäßen Verfahren wird ein Teilstrom des Kreisgases als entsprechend temperierter Gasstrom eingespeist. Aus dem verwendeten Kreisgas wird in den der katalytischen Oxidationsreaktion nachgeschalteten Aufarbeitungsschritten das Produkt abgetrennt, und nach einer Sättigung mit Edukt und einem Vermischen mit der Frischluft bzw. Reaktionsluft wieder in den Prozess mit einer geeigneten Temperatur rückgeführt. Von diesem Kreisgas wird erfindungsgemäß ein entsprechender Teilstrom abgeleitet und unmittelbar unterhalb der Austrittsöffnungen aus der Reaktionszone in den Bereich zwischen unterem Reaktorboden und Auslass eingespeist, wo es sich mit dem Produktgasstrom vermischt.

Erfindungsgemäß wird der temperierte Gasstrom tangential eingespeist.

Das Einspeisen des temperierten Gasstromes erfolgt über in der Wand des erfindungsgemäßen Reaktors angeordnete Einspeiseinrichtungen, welche als Düsen gestaltet sind, die im Bereich der Reaktorwand zwischen dem unteren Rohrboden und dem Auslass des Reaktors angeordnet sind.

Der temperierte Gasstrom wird mittels mehrerer gleichmäßig am Umfang der Wand des Reaktors verteilter Einspeiseinrichtungen in einem Winkel zum Radius tangential eingespeist. Die Winkel können in einem Bereich von 20 bis 60°, bevorzugt von 40 bis 50° zum Radius des Reaktors liegen.

Ein tangentiales Einspeisen führt vorzugsweise zu einer Strömung des temperierten Gasstroms in Umfangsrichtung entlang der Innenwand des des Rohrbündelreaktors, im Bereich der Austrittsöffnung aus der Reaktionszone, vorzugsweise im Bereich der Austrittsöffnungen der Reaktionsrohre. Zusätzlich zu einer Unterdrückung von homogenen Gasphasenreaktionen des Produktes durch die Abkühlung wird somit erreicht, dass der Produktgasstrom aus dem Wandbereich verdrängt wird, wodurch heterogen katalysierte Reaktionen des Produktes an der Wand vermieden werden.

Darüber hinaus bietet das tangentiale Einspeisen des temperierten Gasstroms den Vorteil, dass die Reaktorwand nicht zusätzlich gekühlt werden muss, da eine Energiefreisetzung durch unerwünschte Nachreaktionen vermieden wird.

Die Effektivität der durch das Einspeisen erzeugten Temperierung des Produktgasstroms hängt von einer schnellen Vermischung der Gasströme ab. Durch eine entsprechende Auswahl der verwendeten Einspeiseinrichtungen können hohe Strömungsgeschwindigkeiten des einzuspeisenden Gasstroms erreicht werden, welche eine schnelle Vermischung und somit eine gewünschte rasche Abkühlung erzielen. Die Einspeiseeinrichtungen sind als Düsen ausgebildet. Insbesondere werden mehrere Düsen gleichmäßig am Umfang angeordnet, vorzugsweise 4 bis 8, bevorzugt 6 Düsen. Die Düsen schließen bündig mit der Innenwand der Haube ab. Der Düsenquerschnitt ist so gewählt, dass eine Ausströmgeschwindigkeit des temperierten Gasstroms erreicht werden kann, die im Bereich von 50 bis 100 m/s liegt.

Bei der Ausrichtung der Einspeiseinrichtungen wird eine tangentiale Einspeisrichtung des temperierten Gasstroms erzielt, wodurch eine Gasströmung mit einer tangentialen Strömungskomponente erzeugt wird, welche einen in Umfangsrichtung verlaufenden Drall erzeugt, der über einen turbulenten Strömungszustand einen weiteren positiven Effekt für eine schnelle Vermischung erzielt.

Bedingt durch mögliche Reaktionen und Nebenreaktionen kann die Entstehung von Ablagerungen, insbesondere in Strömungsruhezonen, begünstigt sein. Damit dies weitgehend vermieden werden kann, ist es vorteilhaft, die Innenwand des Reaktors in allen seinen Bereichen, einschließlich der Hauben, glatt auszuführen. Demnach werden die Einspeiseinrichtungen bevorzugt derart gestaltet, dass sie bündig mit der Innenwand des Reaktors abschließen.

Anhand der Zeichnung wird die Erfindung näher erläutert.

Es zeigen:
- Figur 1: eine Darstellung eines zur Herstellung von Glyoxal eingesetzten Rohrbündelreaktors im Längsschnitt;
- Figur 2: eine Darstellung des Rohrbündelreaktors aus Figur 1 im Querschnitt.

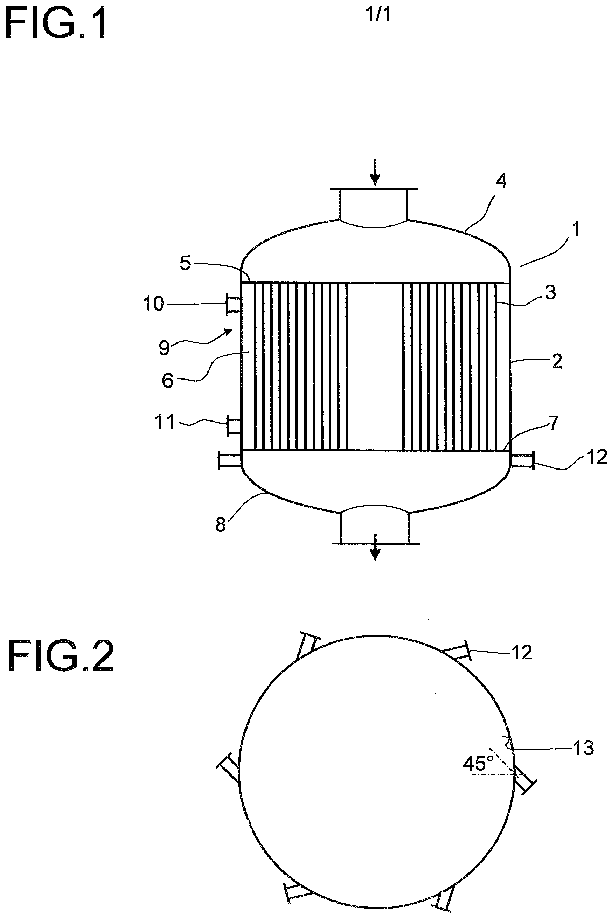

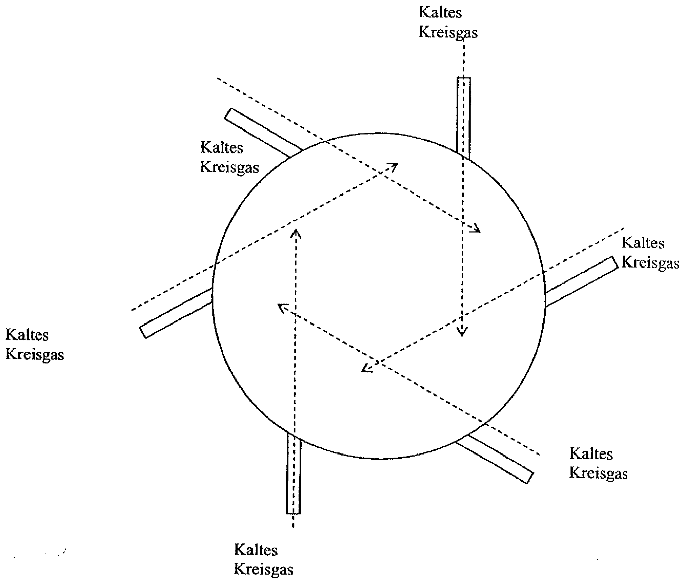

Figur 1 zeigt schematisch dargestellt einen bekannten Rohrbündelreaktor 1, der einen zylindrischen Reaktormantel 2 umfasst, in dem die Reaktionsrohre 3 untergebracht sind. Ein Eduktgemisch, das sich aus Monoethylenglykol im Fall der Herstellung von Glyoxal, Luft, das auf eine gewünschte Temperatur, beispielsweise durch einen Erhitzer, erhitzt wird, und einem Kreisgas zusammensetzt, gelangt in den Rohrbündelreaktor 1, wo es im Bereich der oberen Haube 4 gleichmäßig über den gesamten Reaktorquerschnitt verteilt wird. Die obere Haube 4 wird zum zylindrischen Reaktormantel 2 hin durch einen oberen Rohrboden 5 abgeschlossen. In den Rohrboden 5 münden die Reaktionsrohre 3 des Reaktionsrohrbündels 6. Die Reaktionsrohre 3 sind in ihrem oberen Bereich abdichtend mit dem Rohrboden 5 verschweißt. In den Reaktionsrohren 3 befindet sich das (nicht dargestellte) Katalysatormaterial. In ihrem unteren Bereich sind die Reaktionsrohre 3 mit einem unteren Rohrboden 7 abdichtend verschweißt und münden in eine untere Haube 8 des Rohrbündelreaktors 1. Das Monoethylenglykol-Luftgemisch durchströmt die Reaktionsrohre und wird größtenteils zu Glyoxal umgesetzt.

Das Reaktionsrohrbündel 6 wird durch einen Wärmeaustauschmittelkreislauf, der mit Bezugsziffer 9 bezeichnet ist, temperiert. Dazu wird beispielsweise eine Salzschmelze über Reaktormantelöffnungen 10, 11 in den zylindrischen Mantelabschnitt des Rohrbündelreaktors 1 ein- bzw. ausgeleitet und dort im Längs-, Kreuzquer-, Gegen- oder Gleichstrom an den Reaktionsrohren 3 des Reaktionsrohrbündels 6 vorbeigeführt, um die bei der Oxydehydrierung von Monoethylenglykol entstehende Reaktionswärme abzuführen.

Der heiße Produktgasstrom wird unmittelbar in der unteren Haube 8 durch Einspeisen eines temperierten Gasstroms durch am Umfang vorgesehene Einspeiseinrichtungen 12 abgekühlt.

Die Einspeiseinrichtungen 12 sind bevorzugt in einer Ebene unmittelbar unterhalb des unteren Rohrbodens 7 und somit unmittelbar unterhalb der Austrittsöffnungen der Reaktionsrohre angeordnet.

Figur 2 zeigt einen Querschnitt durch den Rohrbündelreaktor 1 der Figur 1 in einer Ebene, in der die Einspeiseinrichtungen 12 angeordnet sind. In einer bevorzugten Ausführungsform sind die Einspeiseinrichtungen 12 in einem Winkel zum Radius angeordnet, beispielsweise in einem Winkel von 45°. Die Einspeiseinrichtungen 12 verteilen sich regelmäßig an dem Umfang der unteren Haube 8, wobei sie bündig mit der einen Innenwand 13 des Rohrbündelreaktors 8 abschließen.

Mit dem erfindungsgemäßen Verfahren kann durch die entsprechende Temperierung des Produktgasstroms die Ausbeute der Herstellung von Glyoxal von etwa 3 bis 5 % gesteigert werden. Durch die Platz sparende Anordnung der Einspeiseinrichtungen unmittelbar im Bereich des Austritts des Produktgasstroms können die Kosten des Reaktors reduziert werden, die auch von der Länge der Haube des verwendeten Reaktors beeinflusst werden. Die Ausrichtung der Einspeisreinrichtungen, die zu einer tangentialen Strömungskomponente führt, kann sowohl die intensive Kühlung des Produktgasstroms herbeiführen und auch durch die erzeugte Strömungsform entlang der Innenwand dort katalysierte und unselektive Nebenreaktionen unterdrücken.

## Patentansprüche

1. Verfahren zur Durchführung von katalysierten Oxidationsreaktionen von Kohlenwasserstoffen an einem in einem Reaktor angeordneten Katalysator,
**dadurch gekennzeichnet, dass**
• der Produktgasstrom der Oxidationsreaktion unmittelbar nach Austritt aus der Reaktionszone des Reaktors durch Einspeisen eines temperierten Gasstroms gekühlt wird,
• der Reaktor ein Rohrbündelreaktor ist, der Katalysator in den Rohren des Rohrbündelreaktors angeordnet ist und der temperierte Gasstrom unmittelbar unterhalb der Austrittsöffnungen der Reaktionsrohre eingespeist wird,
• der temperierte Gasstrom tangential eingespeist wird,
• der Reaktor eine einen Katalysator enthaltende Reaktionszone aus einer Mehrzahl von mit dem Katalysator gefüllten Reaktionsrohren zwischen einem oberen und einem unteren Rohrboden, einen Einlass für einen Eduktgasstrom in eine obere Reaktorhaube und einen Auslass für einen Produktgasstrom in einer unteren Reaktorhaube umfasst, wobei
• mindestens eine Einspeiseeinrichtung für einen temperierten Gasstrom unmittelbar nach Austritt des Produktgasstroms aus der Reaktionszone im Bereich zwischen dem unteren Rohrboden und dem Auslass des Produktgasstroms an der Reaktorinnenwand vorgesehen ist,
• die Einspeiseeinrichtung am Umfang der Reaktorinnenwand im Bereich der Reaktorhaube zwischen dem unterem Rohrboden und dem Auslass des Produktgasstroms vorgesehen ist,
• die Einspeiseeinrichtung eine tangentiale Einspeisung des temperierten Gasstroms in einem Winkel zum Radius von 20° bis 60° ermöglicht, und
• die Einspeiseeinrichtung mehrere Düsen umfasst.

2. Verfahren nach Anspruch 1, **dadurch gekennzeichnet, dass** die katalysierte Oxidationsreaktion eine Oxydehydrierungsreaktion zur Herstellung von vicinalen Dioxoverbindungen aus entsprechenden Diolen mit Luftsauerstoff ist.

3. Verfahren nach einem der Ansprüche 1 oder 2, **dadurch gekennzeichnet, dass** der temperierte Gasstrom ein Teilstrom eines im Kreislauf geführten Kreisgases des Verfahrens ist.

4. Verfahren nach einem der Ansprüche 1 bis 3, **dadurch gekennzeichnet, dass** die Temperatur des temperierten Gasstroms im Bereich von 30 bis 300°C, bevorzugt im Bereich von 80 bis 200°C und besonders bevorzugt im Bereich von 80 bis 120°C liegt.

5. Verfahren nach einem der Ansprüche 1 bis 4, **dadurch gekennzeichnet, dass** der temperierte Gasstrom im Verhältnis von 1:20 bis 8:10, bevorzugt im Verhältnis von 1:10 bis 1:5 in den Produktgasstrom eingespeist wird.

6. Reaktor zur Durchführung von katalysierten Oxidationsreaktionen nach dem Verfahren gemäß einem der Ansprüche 1 bis 5, umfassend eine einen Katalysator enthaltende Reaktionszone aus einer Mehrzahl von mit dem Katalysator gefüllten Reaktionsrohren zwischen einem oberen und einem unteren Rohrboden, einen Einlass für einen Eduktgasstrom in eine obere Reaktorhaube und einen Auslass für einen Produktgasstrom in einer unteren Reaktorhaube, **dadurch gekennzeichnet, dass**
• mindestens eine Einspeiseeinrichtung für einen temperierten Gasstrom unmittelbar nach Austritt des Produktgasstroms aus der Reaktionszone im Bereich zwischen dem unteren Rohrboden und dem Auslass des Produktgasstroms an der Reaktorinnenwand vorgesehen ist,
• die Einspeiseeinrichtung am Umfang der Reaktorinnenwand im Bereich der Reaktorhaube zwischen dem unterem Rohrboden und dem Auslass des Produktgasstroms vorgesehen ist,
• die Einspeiseeinrichtung eine tangentiale Einspeisung des temperierten Gasstroms in einem Winkel zum Radius von 20° bis 60°, bevorzugt von 40° bis 50° ermöglicht, und
• die Einspeiseeinrichtung mehrere Düsen umfasst.

7. Reaktor nach Anspruch 6, **dadurch gekennzeichnet, dass** eine Vielzahl von ein Katalysatorfestbett enthaltenen Reaktionsrohren, die zwischen einem oberen und einem unteren Rohrboden angeordnet sind, und ein Wärmeaustauschmittelkreislauf in dem Raum zwischen oberem und unterem Rohrboden zur Abführung von Reaktionswärme umfasst sind, und dass mindestens eine Einspeiseeinrichtung für einen temperierten Gasstrom unmittelbar unterhalb der Austrittsöffnungen des Produktgasstromes aus den Reaktionsrohren im Bereich zwischen dem unteren Rohrboden und dem Auslass des Produktgemisches aus dem Reaktor an der Reaktorinnenwand vorgesehen ist.

8. Reaktor nach Anspruch 6, **dadurch gekennzeichnet, dass** die Düsen bündig mit der Innenwand des Reaktors abschließen.

9. Reaktor nach einem der Ansprüche 6 bis 8, **dadurch gekennzeichnet, dass** die Einspeiseeinrichtung den temperierten Gasstrom mit Strömungsgeschwindigkeiten von 50 bis 100 m/s einspeist.

## Claims

1. A process for performing catalyzed oxidation reactions of hydrocarbons over a catalyst disposed in a reactor, wherein
• the product gas stream of the oxidation reaction is cooled immediately after it leaves the reaction zone of the reactor by feeding in a temperature-controlled gas stream,
• the reactor is a tube bundle reactor, the catalyst is disposed in the tubes of the tube bundle reactor and the temperature-controlled gas stream is fed in immediately below the exit orifices of the reaction tubes,
• the temperature-controlled gas stream is fed in tangentially,
the reactor comprises a reaction zone comprising a catalyst and consisting of a plurality of reaction tubes filled with the catalyst between an upper and a lower tube plate, an inlet for a reactant gas stream into an upper reactor hood and an outlet for a product gas stream in a lower reactor hood, where
• at least one feed device for a temperature-controlled gas stream is provided on the inner wall of the reactor immediately after the product gas stream leaves the reaction zone in the region between the lower tube plate and the outlet of the product gas stream,
• the feed device is provided on the circumference of the inner wall of the reactor in the region of the reactor hood between the lower tube plate and the outlet of the product gas stream,
• the feed device enables tangential feeding of the temperature-controlled gas stream at an angle relative to the radius of from 20° to 60°,
• the feed device comprises a plurality of nozzles.

2. The process according to claim 1, wherein the catalyzed oxidation reaction is an oxydehydrogenation reaction for preparing vicinal dioxo compounds from corresponding diols with atmospheric oxygen.

3. The process according to either of claims 1 and 2, wherein the temperature-controlled gas stream is a substream of a circulated cycle gas of the process.

4. The process according to any of claims 1 to 3, wherein the temperature of the temperature-controlled gas stream is in the range from 30 to 300°C, preferably in the range from 80 to 200°C and more preferably in the range from 80 to 120°C.

5. The process according to any of claims 1 to 4, wherein the temperature-controlled gas stream is fed into the product gas stream in a ratio of from 1:20 to 8:10, preferably in a ratio of from 1:10 to 1:5.

6. A reactor for performing catalyzed oxidation reactions by the process according to any of claims 1 to 5, comprising a reaction zone comprising a catalyst and consisting of a plurality of reaction tubes filled with the catalyst between an upper and a lower tube plate, an inlet for a reactant gas stream into an upper reactor hood and an outlet for a product gas stream in a lower reactor hood, wherein
• at least one feed device for a temperature-controlled gas stream is provided on the inner wall of the reactor immediately after the product gas stream leaves the reaction zone in the region between the lower tube plate and the outlet of the product gas stream,
• the feed device is provided on the circumference of the inner wall of the reactor in the region of the reactor hood between the lower tube plate and the outlet of the product gas stream,
• the feed device enables tangential feeding of the temperature-controlled gas stream at an angle relative to the radius of from 20° to 60°, preferably from 40° to 50°, and
• the feed device comprises a plurality of nozzles.

7. The reactor according to claim 6, wherein a multitude of reaction tubes which comprise a fixed catalyst bed and are arranged between an upper and a lower tube plate, and a heat exchange medium circuit are comprised in the space between upper and lower tube plate for removing heat of reaction, and wherein at least one feed device for a temperature-controlled gas stream is provided on the inner wall of the reactor immediately below the exit orifices of the product gas stream from the reaction tubes in the region between the lower tube plate and the outlet of the product mixture from the reactor.

8. The reactor according to claim 6, wherein the nozzles conclude flush with the inner wall of the reactor.

9. The reactor according to any of claims 6 to 8, wherein the feed device feeds in the temperature-controlled gas stream with flow rates of from 50 to 100 m/s.

## Revendications

1. Procédé de réalisation de réactions d'oxydation catalysées d'hydrocarbures sur un catalyseur agencé dans un réacteur,
**caractérisé en ce que**
- le courant gazeux de produits de la réaction d'oxydation est refroidi directement après la sortie de la zone de réaction du réacteur par introduction d'un courant gazeux régulé en température,
- le réacteur est un réacteur à faisceau de tubes, le catalyseur est agencé dans les tubes du réacteur à faisceau de tubes et le courant gazeux régulé en température est introduit directement en dessous des ouvertures de sortie des tubes de réaction,
- le courant gazeux régulé en température est introduit tangentiellement,
- le réacteur comprend une zone de réaction contenant un catalyseur constituée par une pluralité de tubes de réaction remplis avec le catalyseur entre une plaque tubulaire supérieure et une plaque tubulaire inférieure, une entrée pour un courant gazeux de réactifs dans un collecteur de réacteur supérieur et une sortie pour un courant gazeux de produits dans un collecteur de réacteur inférieur,
- au moins un dispositif d'introduction pour un courant gazeux régulé en température étant prévu directement après la sortie du courant gazeux de produits issu de la zone de réaction dans la zone comprise entre la plaque tubulaire inférieure et la sortie du courant gazeux de produits sur la paroi intérieure du réacteur,
- le dispositif d'introduction étant prévu sur la périphérie de la paroi intérieure du réacteur dans la zone du collecteur de réacteur entre la plaque tubulaire inférieure et la sortie du courant gazeux de produits,
- le dispositif d'introduction permettant une introduction tangentielle du courant gazeux régulé en température à un angle par rapport au rayon de 20° à 60°, et
- le dispositif d'introduction comprenant plusieurs buses.

2. Procédé selon la revendication 1, **caractérisé en ce que** la réaction d'oxydation catalysée est une réaction d'oxydéshydrogénation pour la fabrication de composés dioxo vicinaux à partir des diols correspondants avec l'oxygène de l'air.

3. Procédé selon l'une quelconque des revendications 1 ou 2, **caractérisé en ce que** le courant gazeux régulé en température est un courant partiel d'un gaz circulaire du procédé mis en circulation dans un circuit.

4. Procédé selon l'une quelconque des revendications 1 à 3, **caractérisé en ce que** la température du courant gazeux régulé en température se situe dans la plage allant de 30 à 300 °C, de préférence dans la plage allant de 80 à 200 °C et de manière particulièrement préférée dans la plage allant de 80 à 120 °C.

5. Procédé selon l'une quelconque des revendications 1 à 4, **caractérisé en ce que** le courant gazeux régulé en température est introduit dans le courant gazeux de produits en un rapport de 1:20 à 8:10, de préférence en un rapport de 1:10 à 1:5.

6. Réacteur pour la réalisation de réactions d'oxydation catalysées par le procédé selon l'une quelconque des revendications 1 à 5, comprenant une zone de réaction contenant un catalyseur constituée par une pluralité de tubes de réaction remplis avec le catalyseur entre une plaque tubulaire supérieure et une plaque tubulaire inférieure, une entrée pour un courant gazeux de réactifs dans un collecteur de réacteur supérieur et une sortie pour un courant gazeux de produits dans un collecteur de réacteur inférieur, **caractérisé en ce que**
- au moins un dispositif d'introduction pour un courant gazeux régulé en température est prévu directement après la sortie du courant gazeux de produits issu de la zone de réaction dans la zone comprise entre la plaque tubulaire inférieure et la sortie du courant gazeux de produits sur la paroi intérieure du réacteur,
- le dispositif d'introduction est prévu sur la périphérie de la paroi intérieure du réacteur dans la zone du collecteur de réacteur entre la plaque tubulaire inférieure et la sortie du courant gazeux de produits,
- le dispositif d'introduction permet une introduction tangentielle du courant gazeux régulé en température à un angle par rapport au rayon de 20° à 60°, de préférence de 40° à 50°, et
- le dispositif d'introduction comprend plusieurs buses.

7. Réacteur selon la revendication 6, **caractérisé en ce qu'**une pluralité de tubes de réaction contenant un lit catalytique fixe, qui sont agencés entre une plaque tubulaire supérieure et une plaque tubulaire inférieure, et un circuit de moyen d'échange de chaleur dans l'espace compris entre la plaque tubulaire supérieure et la plaque tubulaire inférieure pour la dissipation de la chaleur de réaction sont compris, et **en ce qu'**au moins un dispositif d'introduction pour un courant gazeux régulé en température est prévu directement en dessous des ouvertures de sortie du courant gazeux de produits issu des tubes de réaction dans la zone comprise entre la plaque tubulaire inférieure et la sortie du mélange de produits du réacteur sur la paroi intérieure du réacteur.

8. Réacteur selon la revendication 6, **caractérisé en ce que** les buses finissent en butée contre la paroi intérieure du réacteur.

9. Réacteur selon l'une quelconque des revendications 6 à 8, **caractérisé en ce que** le dispositif d'introduction introduit le courant gazeux régulé en température à des vitesses d'écoulement de 50 à 100 m/s.
